# FASCICULE DE BREVET EUROPEEN

(11) **EP 4 297 832 B1**
(45) Date de publication et mention de la délivrance du brevet: **13.08.2025**
(21) Numéro de dépôt: 22705413.7
(22) Date de dépôt: 23.02.2022
(51) Int. Cl.: A61M 21/00, A61B 5/369, A61B 5/00, G16H 50/20, A61B 5/16, G16H 20/70, G16H 40/67, G16H 40/63

(54) **DISPOSITIF DE MODIFICATION D'UN ÉTAT ÉMOTIONNEL D'UN UTILISATEUR**
VORRICHTUNG ZUR ÄNDERUNG EINES EMOTIONALEN ZUSTANDS EINES BENUTZERS
DEVICE FOR MODIFYING AN EMOTIONAL STATE OF A USER

(30) Priorité: 23.02.2021 FR 2101749
(43) Date de publication de la demande: 03.01.2024
(73) Titulaire: Aphelior, 59800 Lille (FR)
(72) Inventeur: OSLATI, Athénaïs, 60200 Compiegne (FR)
(74) Mandataire: Cornuejols, Georges
(86) Numéro de dépôt international: PCT/EP2022/054518
(87) Numéro de publication internationale: WO 2022/180092

(56) Documents cités:
- WO-A1-2016/070188
- CN-A- 110 947 076
- US-A1- 2015 297 109
- US-A1- 2018 027 347
- US-A1- 2019 060 605

## Description

### Domaine technique de l'invention

La présente invention vise un dispositif et un procédé de modification d'un état émotionnel d'un utilisateur. Elle s'applique, notamment, au domaine de l'amélioration du bien-être et au contrôle de l'état émotionnel d'un individu.

### État de la technique

Le développement des aptitudes cognitives, motrices et sensorielles s'inscrit dans une volonté d'augmenter l'espérance de vie de l'humain. Dans cette recherche de performance et de bien-être, la neuro-technologie est une solution incontournable. Ce domaine de recherche résulte de la convergence entre les neurosciences et l'informatique qui est à l'origine des modifications de schémas et de modèles mentaux.

L'un des objectifs des neuro-technologies est l'augmentation de la performance humaine et l'amélioration de son bien-être. De telles augmentations et améliorations sont possiblement réalisées en modifiant, par exemple, le stress émotionnel de l'humain.

Le stress émotionnel est une réaction naturelle, non pathologique, du corps humain face à des stimuli environnementaux (stresseurs) par exemple. Un tel stress est donc un mécanisme de défense naturel archaïque pouvant s'appliquer à tous les êtres humains, sans être associé à une maladie.

Actuellement, les solutions suivantes sont connues :
- Melomind (marque déposée) : un système composé d'un casque sonore délivrant des sons afin de suivre en direct le niveau de relaxation d'un individu,
- Muse (marque déposée) : un système composé de capteurs EEG (pour « électroencéphalogramme ») mesurent de minuscules champs électriques dans le cerveau, des capteurs de fréquence cardiaque, de respiration et d'oxymétrie,
- Dreem-Urgo (marque déposée) : un système composé d'un capteur de données physiologiques et de capteurs EEG et
- Emotiv (marque déposée) : un système composé d'un capteur EEG et d'écouteurs permettant une indication de du niveau de stress et de distraction de l'utilisateur.
Toutefois, toutes ces solutions présentent les inconvénients suivants :
- Non prise en compte des préférences de l'utilisateur dans les stimuli sonores : Les algorithmes de fourniture de stimuli sonores ne prennent en considération que l'activité cérébrale de l'individu en lui imposant des bruits auxquels il n'est pas habitué, résultant en désintérêt de la part de l'individu voire de rejet des solutions
- Aucune adaptabilité en temps réel : même si ces technologies perçoivent en temps réel les signaux de l'activité cérébrale, les algorithmes utilisés sont incapables de s'adapter aux variations non anticipées des souhaits de l'utilisateur tout au long de ses utilisations. Ils ont été conçus de telle sorte que l'utilisateur va d'un point A au point B par un seul chemin, se basant bien souvent sur : l'activité cérébrale et/ou la physiologie de l'individu (rythme cardiaque).
- Des trajectoires émotionnelles fortement contraintes : Actuellement l'utilisateur ne peut cibler l'état émotionnel qu'il souhaite atteindre. En effet, s'il achète un casque Melomind ou encore Muse, c'est uniquement dans l'objectif de se relaxer. Si certains systèmes comme Emotiv permettent de lire différentes émotions, ils ne permettent pas d'amener l'utilisateur d'une émotion à l'autre. Ces technologies sont souvent unidirectionnelles. Elles partent du stress pour arriver vers un état de relaxation ou encore de concentration. L'inverse n'est donc pas possible.

On connait le document CN 110 947 076 divulguant un dispositif portable intelligent de musique d'ondes cérébrales pour la régulation de l'état mental. On connait également le document US 2019/060 605 qui divulgue un dispositif de modification de l'état cognitif d'un utilisateur. Cependant, aucun de ces dispositifs ne permet une adaptation des fichiers sonores lus selon les choix d'un utilisateur en temps réel.

De plus, on connait le document US 2018/027 347 qui divulgue un système d'analyse du son pour prédire automatiquement l'effet que ces sons auront sur l'utilisateur. Cependant, ce dispositif ne permet pas une combinaison entre une détection de l'état émotionnel de l'utilisateur en temps réel et une adaptation des fichiers sonores lus selon les choix de l'utilisateur en temps réel.

### Objet de l'invention

La présente invention vise à remédier à tout ou partie de ces inconvénients.

À cet effet, selon un premier aspect, la présente invention concerne un dispositif tel que défini dans la revendication 1 et ses revendications dépendantes. Elle vise un dispositif de modification d'un état émotionnel d'un utilisateur, qui comporte :
- un lecteur en temps réel de signaux électroencéphalographiques,
- un module de détermination d'un état émotionnel en fonction d'un signal électroencéphalographique lu,
- un moyen de détermination d'un état émotionnel cible,
- un sélecteur automatique d'une succession d'au moins un fichier sonore, parmi une liste de fichiers sonores préconstituée, en fonction de l'état émotionnel cible déterminé, du signal électroencéphalographique lu et d'au moins un paramètre associé à chaque dit fichier sonore,
- un transducteur électroacoustique configuré pour lire la succession de fichiers sonore sélectionnée,
- un sélecteur secondaire d'un fichier sonore et
- un moyen de mise à jour de la succession en fonction du fichier sonore sélectionné manuellement par le sélecteur secondaire.

Grâce à ces dispositions, un utilisateur peut déterminer volontairement un état émotionnel à atteindre, le dispositif déterminant un vecteur de fichiers sonore optimal et lisant chaque fichier sonore en succession pour adapter graduellement l'état émotionnel de l'utilisateur

L'utilisation d'un lecteur en temps réel de signaux électroencéphalographiques permet une adaptation dynamique de la succession en fonction de son succès sur l'évolution de l'état émotionnel de l'individu ou de ses actions, comme, par exemple, la lecture non prévue d'un fichier sonore nécessitant une mise à jour du vecteur de fichiers sonore.

L'utilisation d'une liste de fichiers sonores préconstituée, notamment produits par des artistes ayant la préférence de l'utilisateur, permet de minimiser le risque de rejet du dispositif par l'utilisateur.

Par ailleurs, l'invention permet à l'utilisateur de sélectionner volontairement un fichier sonore à lire, interrompant la succession sélectionnée, tout en permettant au dispositif d'adapter la succession de fichiers sonores à cette interruption.

Dans des modes de réalisation, le dispositif objet de la présente invention comporte :
- un collecteur d'identifiants de fichiers sonores et
- un classificateur de fichier sonore configuré pour associer, à au moins un identifiant de fichier sonore, un paramètre représentatif d'un état émotionnel favorisé par ledit fichier sonore.

Ces modes de réalisation permettent d'associer à des fichiers sonores d'une liste préconstituée des indicateurs utilisés dans la sélection de la succession de fichiers sonore à lire.

Dans des modes de réalisation, le classificateur est un système d'apprentissage automatique entraîné.

Ces modes de réalisation permettent la classification automatique de fichiers sonores nouvelles, assurant une mise à jour efficace de la liste de fichiers sonores préconstruite.

Dans des modes de réalisation, le système d'apprentissage automatique entraîné est un réseau de neurones supervisé configuré pour recevoir en guise de couche d'entrée des valeurs de paramètres et en guise de couche de sortie des indicateurs d'état émotionnel correspondant à la couche d'entrée.

Dans des modes de réalisation, le classificateur est configuré pour classifier un fichier sonore en attribuant une valeur à au moins une parmi trois caractéristiques :
- la valence,
- l'excitation et
- la dominance,
au moins une caractéristique étant mise en œuvre pour déterminer un état émotionnel favorisé par une piste sonore.

Dans des modes de réalisation, le système d'apprentissage automatique est, de plus, pré-entraîné via l'utilisation d'un jeu de données non spécifique de l'utilisateur.

Grâce à ces dispositions, le système d'apprentissage automatique est pré-entraîné, par exemple, en amont de l'utilisation du dispositif par des données externes. Un entraînement supplémentaire du système d'apprentissage est donc réalisé. Ainsi, le pré-entraînement du système d'apprentissage est renforcé.

Dans des modes de réalisation, au moins un fichier sonore est associé à un indicateur d'un comportement de l'utilisateur vis-à-vis de chaque dit fichier sonore, le sélecteur automatique étant configuré pour sélectionner une succession d'au moins un fichier sonore en fonction d'une valeur dudit indicateur pour au moins un fichier sonore.

Ces modes de réalisation permettent de quantifier la préférence d'un utilisateur vis-à-vis d'un fichier sonore en vue de minimiser le risque de rejet de la succession sélectionnée.

Dans des modes de réalisation, l'indicateur de comportement de l'utilisateur est un paramètre représentatif d'un nombre d'écoutes et/ou d'un nombre d'interruptions de lecture au profit d'une autre piste sonore.

Grâce à ces dispositions, une détermination de l'indicateur de comportement est aisément réalisée.

Dans des modes de réalisation, le sélecteur automatique comporte un filtre de fichier sonore en fonction d'au moins un indicateur d'un comportement de l'utilisateur vis-à-vis d'au moins un fichier sonore, le sélecteur étant configuré pour sélectionner une succession de fichiers sonores parmi une liste de fichiers sonores filtrée par le filtre.

Ces modes de réalisation permettent de quantifier la préférence d'un utilisateur vis-à-vis d'un fichier sonore en vue de minimiser le risque de rejet de la succession sélectionnée.

Selon l'invention, un paramètre utilisé par le sélecteur automatique pour sélectionner une succession de fichiers sonores est un indicateur représentatif d'une valeur d'état émotionnel associée à au moins un fichier sonore.

Dans des modes de réalisation, un paramètre utilisé par le sélecteur automatique pour sélectionner une succession de fichiers sonores est, de plus, un paramètre technique choisi parmi la durée, le mode, la tonalité, le chiffrage de la mesure et le tempo du fichier sonore.

Grâce à ces dispositions, une sélection réalisée par le sélecteur automatique dépend des paramètres techniques inhérents au fichier sonore. Ainsi, l'automatisation du dispositif est renforcée.

Dans des modes de réalisation, selon l'invention, la succession de fichiers sonores est configurée pour présenter un gradient croissant de valeur d'état émotionnel correspondant à l'état émotionnel cible déterminé.

Ces modes de réalisation permettent de déterminer une succession d'intensité, associée à un état émotionnel cible, croissante.

Dans des modes de réalisation, le lecteur en temps réel de signaux électroencéphalographiques est non invasif.

Dans des modes de réalisation, le lecteur est casque de type électroencéphalogramme.

Grâce à ces dispositions, une utilisation du dispositif est facilitée. De plus, une intégrité corporelle de l'utilisateur, lors de l'utilisation du dispositif, est maintenue. Autrement dit, aucune gêne physique n'est présente lors de l'utilisation du dispositif. De plus, lorsque le lecteur est un casque, le dispositif est mobile selon les mouvements de l'utilisateur.

Selon un deuxième aspect non revendiqué, la présente description vise un procédé de modification d'un état émotionnel d'un utilisateur, qui comporte :
- une étape de détermination d'un état émotionnel cible,
puis, de manière itérative, au moins une partie des étapes suivantes :
- une étape de lecture en temps réel de signaux électroencéphalographiques,
- une étape de détermination d'un état émotionnel en fonction d'un signal électroencéphalographique lu,
- une étape de sélection automatique d'une succession d'au moins un fichier sonore, parmi une liste de fichiers sonores préconstituée, en fonction de l'état émotionnel cible déterminé, du signal électroencéphalographique lu et d'au moins un paramètre associé à chaque dit fichier sonore,
- une étape de lecture, par un transducteur électroacoustique, de la succession de fichiers sonore sélectionnée,
- une étape de sélection secondaire d'un fichier sonore et
- une étape de mise à jour de la succession en fonction du fichier sonore sélectionné manuellement par le sélecteur secondaire.

Les avantages, buts et caractéristiques particulières du procédé objet de la présente invention étant identiques à ceux du dispositif objet de la présente invention, ils ne sont pas rappelés ici.

### Brève description des figures

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels :
- la figure 1 représente, schématiquement, un premier mode de réalisation particulier du dispositif objet de la présente invention,
- la figure 2 représente, schématiquement, un deuxième mode de réalisation particulier du dispositif objet de la présente invention,
- la figure 3 représente, schématiquement et sous forme d'un logigramme, une succession d'étapes particulière du procédé objet de la présente invention,
- la figure 4 représente schématiquement, une distribution d'un échantillon de fichiers musicaux en matière de nature énergisante, répartis entre des valeurs de 0,1 et 1,
- la figure 5 représente schématiquement, une distribution d'un échantillon de fichiers musicaux en matière de nature dansante, répartis entre des valeurs de 0,1 et 1,

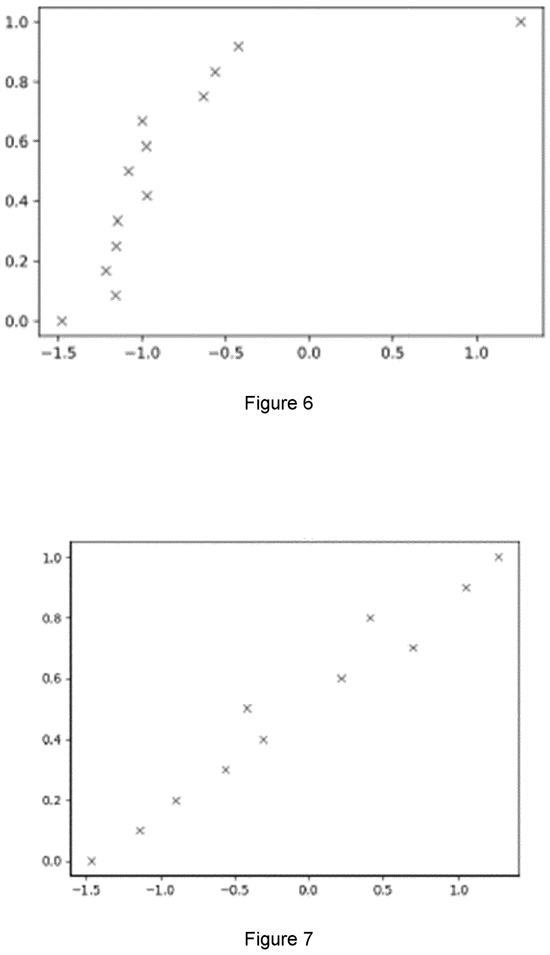

- la figure 6 représente schématiquement, une distribution de fichiers sonores à lire correspondant à un vecteur logarithmique déterminé de modification d'état émotionnel,
- la figure 7 représente schématiquement, une distribution de fichiers sonores à lire correspondant à un vecteur linéaire déterminé de modification d'état émotionnel,
- la figure 8 représente, schématiquement, une distribution de valeurs de nature acoustique de fichiers sonores correspondant à des états émotionnels distincts et
- la figure 9 représente, schématiquement, une distribution de valeurs de volume sonore de fichiers sonores correspondant à des états émotionnels distincts.

### Description des modes de réalisation

La présente description est donnée à titre non limitatif, chaque caractéristique d'un mode de réalisation pouvant être combinée à toute autre caractéristique de tout autre mode de réalisation de manière avantageuse.

On note dès à présent que les figures ne sont pas à l'échelle.

On appelle « état émotionnel », les résultats de l'interaction de facteurs subjectifs et objectifs, réalisés par des systèmes neuronaux ou endocriniens, qui peuvent :
- induire des expériences telles que des sentiments d'éveil, de plaisir ou de déplaisir ;
- générer des processus cognitifs tels que des réorientations pertinentes sur le plan perceptif, des évaluations, des étiquetages ;
- activer des ajustements physiologiques globaux ;
- induire des comportements qui sont, le plus souvent, expressifs, dirigés vers un but et adaptatifs.

Un tel état émotionnel est, par exemple, pour un humain :
- Le stress (difficulté à se concentrer, cheminement de pensée chaotique et perturbé par la source du stress, excitation, peur),
- La relaxation (état calme, absence ou faible activité de réflexion, les idées s'enchaînent de façon lente),
- La concentration (activité cérébrale intense, non-réceptivité aux signaux extérieurs) et
- L'intérêt ou l'engagement (mesure le niveau d'immersion dans un stimulus quelconque. L'engagement peut augmenter lors de l'immersion dans des stimuli qui peuvent être de nature positive ou négative. Il a été constaté dans une recherche expérimentale sur le cerveau que l'engagement diminue dans les processus cognitifs ennuyeux, banaux et automatiques).

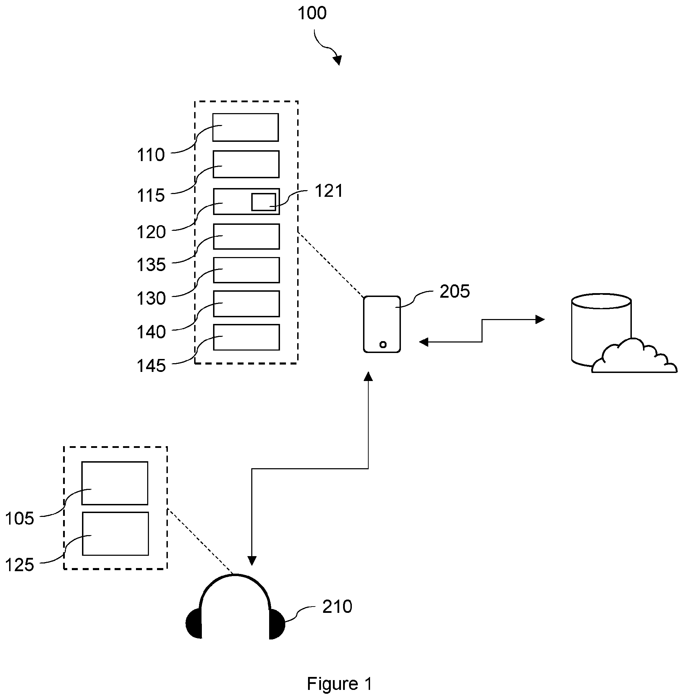

On observe, sur la figure 1, qui n'est pas à l'échelle, une vue schématique d'un mode de réalisation du dispositif 100 objet de la présente invention. Ce dispositif 100 de modification d'un état émotionnel d'un utilisateur comporte :
- un lecteur 105 en temps réel de signaux électroencéphalographiques,
- un module 110 de détermination d'un état émotionnel en fonction d'un signal électroencéphalographique lu,
- un moyen 115 de détermination d'un état émotionnel cible,
- un sélecteur 120 automatique d'une succession d'au moins un fichier sonore, parmi une liste de fichiers sonores préconstituée, en fonction de l'état émotionnel cible déterminé, du signal électroencéphalographique lu et d'au moins un paramètre associé à chaque dit fichier sonore et
- un transducteur 125 électroacoustique configuré pour lire la succession de fichiers sonore sélectionnée.

Le lecteur 105 est, par exemple, un casque de type électroencéphalogramme muni d'écouteurs agissant en tant que transducteur 125 électroacoustique. Le type d'électroencéphalogramme considéré peut-être de tout type connu d'une personne compétente dans le métier des neuro-technologies. Préférentiellement, un électroencéphalogramme de type non-invasif est mis en œuvre.

Un électroencéphalogramme a pour fonction la capture de signaux électriques résultants de la sommation des potentiels post-synaptiques synchrones issus d'un grand nombre de neurones. Un tel signal peut être représentatif d'une activité neurophysiologique de l'individu portant le lecteur 105 et, ainsi, d'un état émotionnel de cet individu.

L'acquisition est par exemple faite à partir de sept électrodes sèches placées sur le crâne d'un utilisateur, préférentiellement en positions A1, T3, C3, CZ, C4, T4, A2, T5 et T6 selon le système 10-20. Ces électrodes mesurent la différence de potentiel (en Volts) entre les différents emplacements et "la masse", placée sur l'oreille la plus proche.

Le choix du positionnement ces électrodes est principalement lié à la géométrie du casque et au confort d'utilisation, mais aussi à la sélection de certains points moins soumis aux artéfacts moteurs (clignements de paupières, reniflements, etc).
Les électrodes sont reliées à une carte numérique. Cette carte est configurée pour transmettre le signal au module 110 de détermination ou à un ordinateur, par exemple via Bluetooth à un récepteur USB branché sur l'ordinateur.

La durée d'échantillonnage choisie est par exemple de 5 secondes (toutes les 5 secondes, on récupère le signal des 5 dernières secondes passées). Cette valeur pourrait être augmentée à 8 secondes, voire 10 secondes, 5, 8 et 10 étant les durées d'échantillons permettant la meilleure inférence des émotions.

À partir du signal brut, l'intensité des différentes fréquences peut être calculée par transformée de Fourier. Un premier pré-traitement du signal peut être appliqué pour supprimer les fréquences proches de 50 Hz (49 à 51 Hz) ou de 60 Hz (59 à 61 Hz) qui sont intensément parasitées en présence d'appareils électriques branchés sur le secteur à proximité de l'appareil d'enregistrement (le casque).

Un second filtre passe-bande peut être appliqué pour ne conserver que les fréquences dans la gamme 2 à 58 Hz, afin d'éliminer le bruit parasite de basse fréquence, et les bandes gamma haute fréquence (60 Hz et plus) que le Bluetooth ne nous permet pas de décrire proprement.

Les deux filtres utilisés sont, par exemple, de type Butterworth d'ordre 5.
Le module 110 de détermination est, par exemple, un logiciel informatique exécuté par un circuit électronique de calcul. Ce module 110 de détermination est configuré, par exemple, pour déterminer un état émotionnel de l'individu de la manière suivante :
On choisit comme modèle pour décrire les émotions le système à trois variables et couramment utilisé appelé modèle "VAD" pour les axes de Valence, Arousal (traduit par « excitation »), et Dominance en anglais.

La valence décrit le caractère négatif, neutre, ou positif associé à une émotion.

L'excitation mesure le caractère passif, neutre ou actif de l'état émotionnel décrit.

La dominance décrit le caractère affirmé ou soumis de l'état émotionnel décrit. Cet axe permet par exemple de discriminer la rage de la peur (qui sont toutes deux caractérisées par une valence faible et une excitation forte), ou encore la relaxation de la joie.

On se donne, pour chaque axe, 3 valeurs discrètes possibles :
Valence : -1 pour négative, 0 pour neutre, 1 pour positive,
Arousal : -1 pour passive, 0 pour neutre, 1 pour active et
Dominance : -1 pour basse, 0 pour moyenne, 1 pour haute.

On attribue les étiquettes suivantes aux triplets de valeurs V-A-D :
- Excité : (1, 1, 1)
- Heureux : (1,0,1)
- Content : (1,0,0)
- Relaxé, Détendu : (1,-1,0)
- Calme : (0,-1,0)
- Triste, Déprimé : (-1, -1, -1)
- Déstressé : (0,1,0)
- Neutre : (0,0,0)
- Chagrin profond : (-1,0,-1)

D'autres descripteurs émotionnels peuvent être utilisés en plus des valeurs VAD, car faciles à détecter et reconnaître à partir d'enregistrements cérébraux, telles la relaxation et la concentration.

Chaque piste sonore peut être placée selon ces coordonnées de sorte à calculer un vecteur de proche en proche permettant, en partant de coordonnées déterminées, d'attendre d'autres coordonnées correspondant à un état émotionnel cible.

Le module 110 de détermination peut être mis en œuvre localement ou à distance et accessible via un réseau de données. Par exemple, le module 110 de détermination peut être mis en œuvre sur un téléphone intelligent connecté au lecteur 105 de manière filaire ou, préférentiellement, sans-fil.

Le moyen 115 de détermination d'un état émotionnel cible est, par exemple, une interface homme-machine (par exemple de type interface graphique associée à un périphérique de saisie) ou une interface logicielle (par exemple de type API, pour Application Programming Interface, traduit par « Interface de programmation d'application »). Ce moyen 115 de détermination est configuré pour recevoir, en entrée, un signal variable entre plusieurs états émotionnels possibles.
Ces états émotionnels peuvent être prédéterminés, c'est-à-dire former une liste finie de possibilités parmi laquelle une interface mise en œuvre sélectionne.
Ces états émotionnels peuvent être déterminés en fonction du contenu d'une saisie réalisée sur une interface. Par exemple, une interface homme-machine permet la libre saisie de caractères alphanumériques représentatifs d'un langage humain, un utilisateur du dispositif 100 entrant des mots-clés descripteurs d'un état émotionnel à atteindre, moyen 115 de détermination étant configuré pour associer à ces mots-clés des états émotionnels définis.

Le moyen 115 de détermination peut être mis en œuvre localement ou à distance et accessible via un réseau de données. Par exemple, le moyen 115 de détermination peut être mis en œuvre sur un téléphone intelligent associé au module 110 de détermination.

Le sélecteur 120 automatique est, par exemple, un logiciel informatique exécuté par un circuit électronique de calcul. Le sélecteur 120 automatique est configuré, par exemple, pour exécuter un algorithme mesurant une distance entre l'état émotionnel lu d'un utilisateur du dispositif 100 et l'état cible déterminé par le moyen 115 de détermination. En fonction de la distance ainsi mesurée, une succession d'au moins un fichier sonore est sélectionnée selon au moins un paramètre associé à au moins un dit fichier sonore.

Un tel paramètre peut être, par exemple :
- un paramètre technique, tel que la durée, le mode, la tonalité, le chiffrage de la mesure ou le tempo du fichier sonore ou
- un paramètre acoustique ou psychoacoustique représentatif de :
   - la nature acoustique du fichier sonore, c'est-à-dire l'utilisation ou non d'instruments électroniques et/ou la proportion d'une telle utilisation,
   - la nature énergisante, ou vivifiante, du fichier sonore, c'est-à-dire une mesure perceptive de l'intensité et de l'activité - les caractéristiques perceptuelles contribuant à cet attribut comprennent la gamme dynamique, l'intensité sonore perçue, le timbre, le taux d'apparition et l'entropie générale,
   - la nature instrumentale du fichier sonore, c'est-à-dire l'utilisation ou non de la voix sur ledit fichier sonore,
   - la nature dansante du fichier sonore, mesuré par exemple en fonction du tempo, de la stabilité du rythme, de la force du battement et de la régularité générale du fichier audio,
   - la valence du fichier sonore, c'est-à-dire la positivité du fichier sonore,
   - la nature de l'enregistrement du fichier sonore, c'est-à-dire l'enregistrement studio ou lors d'une prestation en direct du fichier sonore,
   - la nature de densité de paroles, c'est-à-dire la proportion de mots et de musique dans un fichier sonore et/ou
   - l'intensité du fichier sonore, c'est-à-dire l'intensité moyenne, mesurée en décibels, du fichier sonore.

Chacun de ces paramètres peut être directement associé à un état émotionnel et ainsi, en fonction de l'état émotionnel cible, être candidat à l'inclusion dans la succession de fichiers sonores.

L'association entre valeurs pour ces paramètres et état émotionnels (via, par exemple, un profil V-A-D) peut être réalisée, par exemple, par la mise en œuvre d'un algorithme d'apprentissage, obtenu de manière similaire au jeu de données IADS-E du centre d'étude des émotions et de l'attention de l'université de Floride.

Alternativement, un système expert peut être mis en œuvre, associant des valeurs particulières du profil V-A-D à un état émotionnel. Un exemple d'une telle mise en œuvre est fourni plus haut.

Dans un mode simplifié, la nature énergisante et la nature dansante sont associées à l'excitation, le mode et la valence avec la valence et l'intensité avec la dominance.

Pour chaque échantillon, on construit ensuite, par exemple, un modèle statistique de chaque descripteur acoustique. Le modèle choisi est, par exemple, une mixture gaussienne, c'est-à-dire un ensemble pondéré d'une à cinq courbes de Gauss ("courbes en cloche") dont les moyennes et écarts-types sont enregistrés, et les poids associés à chaque Gaussienne aussi. Le modèle mixte Gaussien obtenu décrit une courbe de densité de probabilité, qui associe à chaque valeur du paramètre acoustique considéré la probabilité d'être observée pour une piste audio du groupe donné (haute ou basse valence, haute ou basse arousal, haute ou basse domination).

On a donc obtenu une approximation de la probabilité qu'une piste audio de caractéristiques acoustiques données soit dans chaque cadran de l'espace VAD.

On calcule la moyenne, sur chaque axe et pour chaque piste audio, de la probabilité d'appartenir au cadran positif et négatif. On obtient alors les coordonnées d'un fichier sonore en question dans l'espace VAD. Cette position dans l'espace VAD sera lue lors de la détermination des pistes audio à ajouter à une liste de lecture.

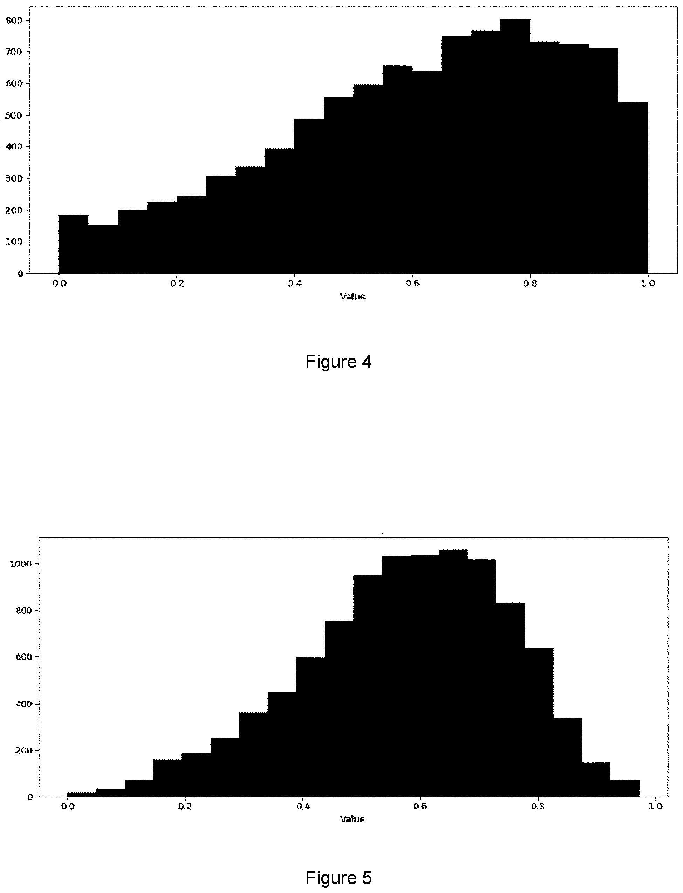

On observe, en figure 4, schématiquement, une distribution d'un échantillon de fichiers musicaux en matière de nature énergisante, répartis entre des valeurs de 0,1 et 1.

On observe, en figure 5, schématiquement, une distribution d'un échantillon de fichiers musicaux en matière de nature dansante, répartis entre des valeurs de 0,1 et 1.

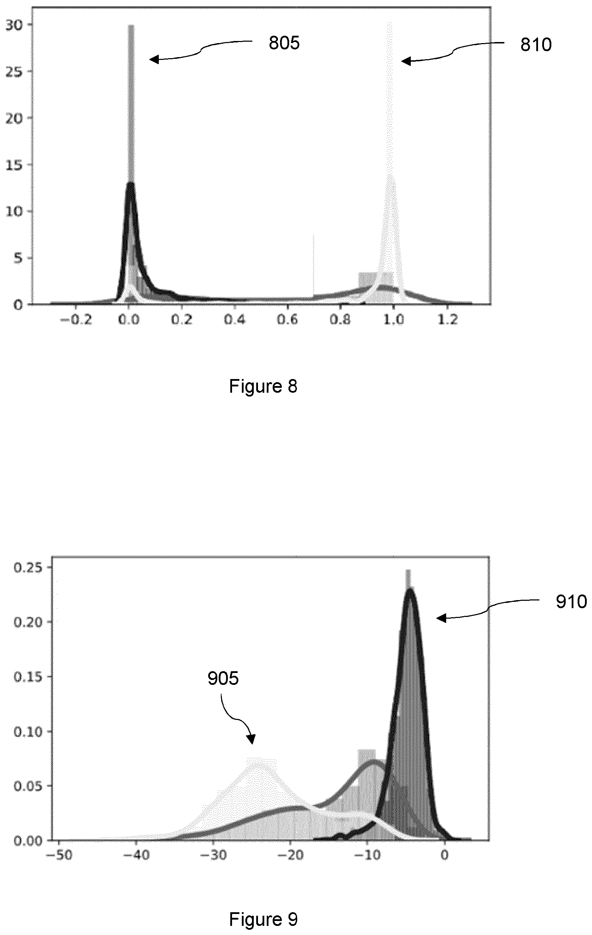

On observe, en figure 8, schématiquement, une distribution de la quantification de la nature acoustique d'un échantillon de fichiers sonores correspondant à, notamment, deux états distincts :
- un état émotionnel calme 805 et
- un état émotionnel de concentration 810.

On observe, en figure 9, schématiquement, une distribution de la quantification du volume sonore d'un échantillon de fichiers sonores correspondant à, notamment, deux états distincts :
- un état émotionnel de concentration 905 et
- un état émotionnel d'intense activité physique 910.

Préférentiellement, un paramètre utilisé par le sélecteur 120 automatique pour sélectionner une succession de fichiers sonores est un indicateur représentatif d'une valeur d'état émotionnel associée à au moins un fichier sonore. Chaque fichier sonore est alors associé à un vecteur de quantification de l'impact pour au moins un état émotionnel. Par exemple, un fichier sonore peut avoir une première valeur correspondant à l'impact de ce fichier sonore sur un niveau de stress d'un auditeur et une deuxième valeur correspondant à l'impact de ce fichier sonore sur un niveau de relaxation d'un auditeur.

Préférentiellement, la succession de fichiers sonores est configurée pour présenter un gradient croissant de valeur d'état émotionnel correspondant à l'état émotionnel cible déterminé.

Autrement dit, les états émotionnels actuels et cibles déterminées sont décrits par des coordonnées sur des axes que constituent tout ou partie des paramètres listés ci-dessus, dans un espace multi-dimensionnel.
Un tel vecteur peut être construit à partir d'au moins un des paramètres décrits ci-dessus.

Un tel vecteur, dans un espace dimensionnel déterminé, peut correspondre à une fonction affine ou logarithmique.

Selon un premier algorithme, une trajectoire théorique en ligne droite entre les deux points dans l'espace VAD est d'abord calculée. Elle ne correspond pas encore physiquement à une liste de fichiers sonores. Puis, l'algorithme échantillonne des points régulièrement espacés le long de cette ligne théorique (en fonction du nombre de fichiers sonores souhaités, lui-même fonction de la durée de la liste de lecture souhaitée). Enfin, on sélectionne les fichiers sonores de la base de données dont les coordonnées dans cet espace sont les plus proches de chacun des points théoriques, ce qui aboutit à une liste ordonnée de fichiers sonores.

Selon un deuxième algorithme, la sélection des pistes audio se fait de façon itérative, en recherchant systématiquement le fichier le plus proche du point médian entre les bornes de l'intervalle de recherche. On commence par calculer le point médian théorique entre les deux points (états actuel et ciblé). On détermine ensuite le fichier sonore dans la base de données la plus proche de ce point dans l'espace VAD, à l'aide de ses coordonnées. Ce fichier permet de couper l'intervalle en deux et de produire deux nouveaux intervalles, sur lesquels on répète la procédure, jusqu'à, optionnellement, l'obtention d'un nombre maximal de pistes audio.

La trajectoire finale obtenue est moins linéaire qu'avec l'algorithme 1, mais permet des transitions plus fluides entre les pistes.

Le sélecteur 120 automatique peut être mis en œuvre localement ou à distance et accessible via un réseau de données. Par exemple, le sélecteur 120 automatique peut être mis en œuvre sur un téléphone intelligent associé aux moyens 115 de détermination et au module 110 de détermination.

La succession de fichiers sonores est envoyée à un transducteur 125 électroacoustique. Cet envoi peut être réalisé par l'intermédiaire de la carte-mère ou d'une carte-son d'un téléphone intelligent interfacé avec le sélecteur 120 automatique.

Dans des modes de réalisations particuliers, tel que celui représenté sur la figure 1, le dispositif 100 objet de la présente invention comporte :
- un collecteur 140 d'identifiants de fichiers sonores et
- un classificateur 145 de fichier sonore configuré pour associer, à au moins un identifiant de fichier sonore, un paramètre représentatif d'un état émotionnel favorisé par ledit fichier sonore.

Le collecteur 140 d'identifiants est, par exemple, un logiciel informatique exécuté par un circuit électronique de calcul. Ce collecteur 140 est, par exemple, configuré pour collecter les identifiants de fichiers sonores dont la lecture est commandée par au moins un utilisateur associé au dispositif 100 à partir d'une application tierce de lecture de fichiers sonores.
Dans des variantes, le collecteur 140 d'identifiants est un logiciel de lecture de métadonnées d'identifiants de fichiers sonores stockés dans un stockage informatique local ou distant.

Le collecteur 140 d'identifiants peut être mis en œuvre localement ou à distance et accessible via un réseau de données.

Le classificateur 145 est, par exemple, un logiciel informatique exécuté par un circuit électronique de calcul. Ce classificateur 145 est configuré pour, à partir de paramètres des fichiers sonores, tels que décrits ci-dessus, attribuer une valeur quantitative de l'impact du fichier sonore sur l'état émotionnel d'un auditeur du fichier sonore.

Dans des modes de réalisation particuliers, le classificateur 145 est un système d'apprentissage automatique entraîné. Un tel classificateur 145 peut être, par exemple, un algorithme d'apprentissage automatique supervisé ou non et de type apprentissage profond ou non.

Par exemple, un tel système d'apprentissage automatique est un dispositif à réseau de neurones supervisé configuré pour recevoir en guise de couche d'entrée des valeurs de paramètres, tels que mentionnés ci-dessus, et en guise de couche de sortie des indicateurs d'état émotionnel correspondant à la couche d'entrée.

Dans des modes de réalisation, le classificateur 145 est configuré pour classifier un fichier sonore en attribuant une valeur à au moins une parmi trois caractéristiques :
- la valence,
- l'excitation et
- la dominance,
au moins une caractéristique étant mise en œuvre pour déterminer un état émotionnel favorisé par une piste sonore. Optionnellement, la concentration et/ou la relaxation peut recevoir une valeur, via le classificateur 145 ou un autre mécanisme d'attribution.

Dans un exemple de mise en œuvre consistant à réaliser un tel classificateur 145, un programme informatique permet à l'utilisateur de signaler lorsqu'il ressent profondément, dans son corps, qu'il est dans l'un des états émotionnels listés plus haut. S'il confirme son état, l'échantillon enregistré est envoyé au modèle de classification qui renforce son apprentissage.

L'utilisation fréquente de cet outil de renforcement est nécessaire pour que le modèle apprenne correctement. Avant l'utilisation suffisante de cet outil par l'utilisateur (plusieurs dizaines de fois, avec représentation de toutes les émotions), la performance du modèle passe d'aléatoire, à mauvaise, puis médiocre, et enfin acceptable. Il est possible qu'un classificateur 145 pré-entraîné soit mis en œuvre via l'utilisation d'un jeu de données non spécifique de l'utilisateur, pour démarrer avec une performance correcte.

On entraîne le modèle à reconnaître une émotion non pas à partir du signal brut, mais à partir de transformations de celui-ci, qu'on appelle des caractéristiques qui sont calculées à partir d'un échantillon enregistré pendant un temps t, sur les 7 canaux listés plus haut :
Pour chaque canal :
- les caractéristiques spatiales : l'intensité des bandes de fréquences alpha [7-13 Hz], bêta [14-30 Hz] et gamma [31-90Hz] obtenues par transformée de Fourier, et l'entropie différentielle et
- les caractéristiques temporelles : l'entropie approximative, l'entropie d'échantillon, et la dimension fractale.

En prenant en compte tous les canaux :
- les caractéristiques multi-échelle (Multi-Scale-Entropy features) et
- l'entropie de Renyi (ou plus précisément, une estimation non-paramétrique de l'entropie de Renyi).

Un algorithme de classification peut être une méthode d'ensemble (une méthode qui fait la moyenne entre la prédiction de plusieurs classifieurs) appelée "Random Forest", les classificateurs utilisés étant des arbres de décision. On utilise une population de cent arbres de décision, par exemple.
Un arbre de décision est une série de règles qui utilisent des seuils sur les valeurs des caractéristiques.

La phase d'entraînement de l'algorithme consiste à faire varier ces seuils jusqu'à obtenir une qualité de prédiction suffisante. Chaque nouvel échantillon obtenu dont on connaît l'état émotionnel associé permet d'affiner un peu plus les seuils.
La performance des modèles varie d'un individu à l'autre et ne peut pas être estimée de façon générale. La littérature scientifique annonce des moyennes oscillantes entre 60% et 85% de prédictions correctes, selon les individus.

Ainsi, le programme informatique qui constitue le moyen d'entraînement effectue, les étapes suivantes :
- réceptionner le signal brut de la part de du casque EEG (échantillon de 5 secondes),
- calculer les caractéristiques du signal,
- estimer les valeurs de valence, arousal, et domination de l'état émotionnel actuel de l'utilisateur, en utilisant une moyenne des prédictions des arbres de décision.
- transformer les coordonnées (V, A, D) obtenues en un pourcentage de chaque émotion dont l'étiquette est connue, par un calcul de distance euclidienne à la position de ces émotions connues dans l'espace VAD et à une normalisation de l'inverse de ces distances,
- si l'utilisateur signale, via un périphérique d'entrée (clavier, écran tactile), qu'il est dans un état émotionnel connu, lui proposer d'utiliser le dernier échantillon pour participer à l'entraînement du modèle et s'il valide, ajuster les seuils des arbres de décision à l'aide de ce nouvel échantillon et
- attendre la réception d'un nouvel échantillon.

À l'arrêt du programme d'entraînement, la population d'arbres de décision est sauvegardée, pour être chargée au prochain démarrage.

Dans des modes de réalisation particuliers, au moins un fichier sonore est associé à un indicateur d'un comportement de l'utilisateur vis-à-vis de chaque dit fichier sonore, le sélecteur 120 automatique étant configuré pour sélectionner une succession d'au moins un fichier sonore en fonction d'une valeur dudit indicateur pour au moins un fichier sonore.

Un tel indicateur de comportement est, par exemple, un paramètre représentatif d'un nombre d'écoutes, d'un nombre d'interruptions de lecture au profit d'une autre piste sonore ou tout autre paramètre représentatif d'une volonté de l'utilisateur. Cet indicateur de comportement peut être mis en œuvre dans la sélection de la succession de fichiers sonores, par exemple en affectant une pondération moindre aux fichiers sonores candidats dont le nombre d'interruptions de lecture est plus élevé que la moyenne, traduisant une insatisfaction de l'utilisateur lors de la lecture desdits fichiers.

Dans des modes de réalisation, au moins un paramètre utilisé par le sélecteur 120 automatique est un paramètre représentatif d'une proximité musicale entre fichiers sonores. Une telle proximité musicale peut être établie, par exemple, en fonction d'une métadonnée représentative d'un genre musical ou en fonction de l'un des paramètres décrits ci-dessus, en regard du sélecteur 120 automatique.

Une proximité musicale est déterminée en fonction des distances euclidiennes dans l'espace des paramètres (normalisés par leurs unités) exemplifiés ci-dessus.

Dans des modes de réalisations particuliers, tel que celui représenté sur la figure 1, le sélecteur 120 automatique comporte un filtre 121 de fichier sonore en fonction d'au moins un indicateur d'un comportement de l'utilisateur vis-à-vis d'au moins un fichier sonore, le sélecteur étant configuré pour sélectionner une succession de fichiers sonores parmi une liste de fichiers sonores filtrée par le filtre.

Un tel filtre 121 est, par exemple, un filtre logiciel permettant d'établir un échantillon de fichiers sonores candidats à la sélection dans la succession de fichiers sonores en amont de la sélection effective.

Dans des modes de réalisations particuliers, tels que celui représenté sur la figure 1, le dispositif 100 objet de la présente invention comporte, de plus, un sélecteur 130 secondaire d'un fichier sonore et un moyen 135 de mise à jour de la succession en fonction du fichier sonore sélectionné manuellement par le sélecteur secondaire. Le sélecteur 130 secondaire est, par exemple, une interface homme-machine (par exemple de type interface graphique associée à un périphérique de saisie) ou une interface logicielle (par exemple de type API). Ce sélecteur 130 secondaire est configuré pour recevoir, en entrée, un identifiant de fichier sonore à lire. Une variante d'un tel sélecteur 130 secondaire est, par exemple, un écran tactile associé à une interface utilisateur graphique (« GUI », en anglais, pour « Graphic User Interface ») permettant la saisie d'un identifiant de fichier sonore.

Ce sélecteur 130 permet à un utilisateur de forcer la lecture d'un fichier sonore, indépendamment de l'effet bénéfique ou négatif de ce fichier sonore sur l'état émotionnel cible déterminé.

Toutefois, la quantification de cet effet bénéfique ou négatif permet au moyen 115 de détermination de déterminer une nouvelle succession de fichiers sonores permettant l'atteinte de l'état émotionnel cible ultérieur en fonction de la déviation causée par la lecture du fichier sonore sélectionné par le sélecteur 130 secondaire.

Le sélecteur 130 secondaire peut être mis en œuvre localement ou à distance et accessible via un réseau de données.

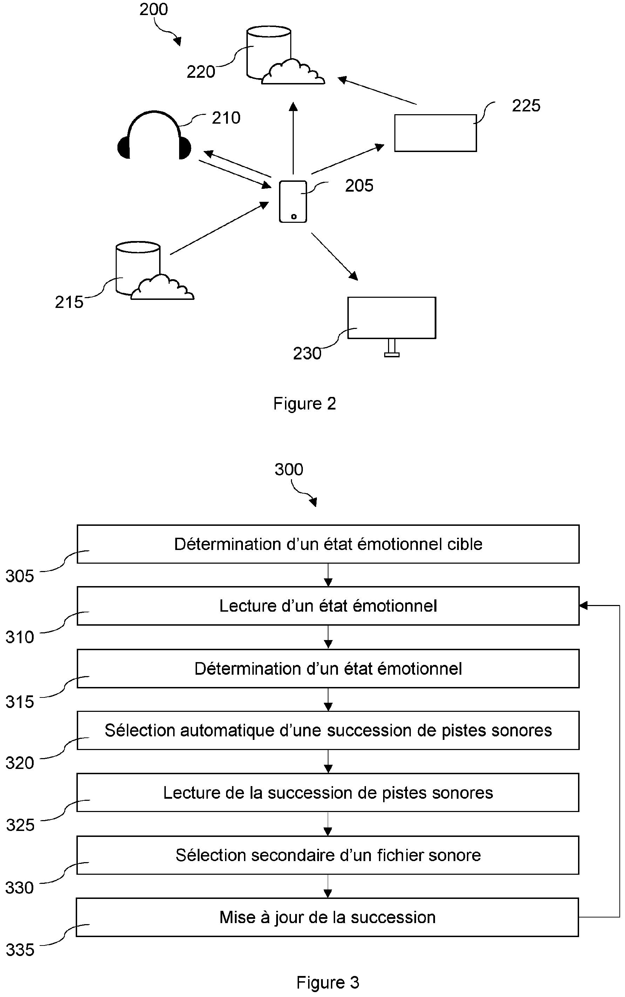

On observe, sur la figure 2, schématiquement, un mode de réalisation particulier d'un écosystème technique 200 permettant la mise en œuvre du dispositif 100 objet de la présente invention. Cet écosystème technique 200 comporte :
- un terminal 205 central, de type circuit électronique de calcul configuré pour exécuter des séquences d'instructions algorithmiques sous forme logicielle, tel par exemple un ordinateur, un serveur ou un téléphone intelligent,
- une base de données 215 de fichiers audios accessibles depuis le terminal central 205,
- un casque 210 EEG muni d'écouteurs permettant la restitution de fichiers audio, connecté au terminal 205 central de sorte à :
- recevoir un fichier audio à lire (ou une localisation de ressource à lire sur un réseau de données) et
- envoyer audit terminal 205 un indicateur représentatif d'un signal EEG lu, ledit terminal 205 déterminant localement un état émotionnel du porteur du casque 210 ou transmettant cet indicateur pour détermination à distance, via l'utilisation d'un serveur informatique par exemple,
- une base de données 220 de classification d'états émotionnels associés à des fichiers sonores, associée à une ressource informatique de calcul (non référencée) configurée pour exécuter un algorithme de classification, cette base de données 220 pouvant être nourrie à fur et à mesure des lectures de fichiers audio par le terminal 205 central ou directement connecté à la base 215 de données de fichiers audio,
- une interface 225 d'accès à la base de données 220 de classification, par le terminal central 205 (ou une autre ressource de calcul), configuré pour produire, en fonction d'un état émotionnel cible déterminé, une liste de fichiers sonores candidats à l'inclusion dans la succession à produire pour modifier l'état émotionnel de l'utilisateur, ou alternativement directement la succession de fichiers sonores à lire et
- optionnellement, une interface 230 de suivi et de contrôle permettant l'affichage de statistiques et d'une interface de paramétrage du dispositif 100.

On observe, sur la figure 3, schématiquement, un mode de réalisation particulier du procédé 300 objet de la présente invention. Ce procédé 300 de modification d'un état émotionnel d'un utilisateur, comporte :
- une étape 305 de détermination d'un état émotionnel cible,
puis, de manière itérative, au moins une partie des étapes suivantes :
- une étape 310 de lecture en temps réel de signaux électroencéphalographiques,
- une étape 315 de détermination d'un état émotionnel en fonction d'un signal électroencéphalographique lu,
- une étape 320 de sélection automatique d'une succession d'au moins un fichier sonore, parmi une liste de fichiers sonores préconstituée, en fonction de l'état émotionnel cible déterminé, du signal électroencéphalographique lu et d'au moins un paramètre associé à chaque dit fichier sonore et
- une étape 325 de lecture, par un transducteur électroacoustique, de la succession de fichiers sonore sélectionnée.

Dans des modes de réalisation, tel que celui représenté en figure 3, le procédé 300 comporte, de plus :
- une étape 330 de sélection secondaire d'un fichier sonore et
- une étape 335 de mise à jour de la succession en fonction du fichier sonore sélectionné manuellement par le sélecteur secondaire.

Des exemples de mise en œuvre des étapes du procédé 300 sont décrits au regard des moyens correspondants, tels que décrits en regard des figures 1 et 2.

Préférentiellement, les moyens du dispositif 100 et de l'écosystème technique 200 sont configurés pour mettre en œuvre les étapes du procédé 300 et leurs modes de réalisation tels qu'exposés ci-dessus et le procédé 300 ainsi que ses différents modes de réalisation peuvent être mis en œuvre par les moyens du dispositif 100 et/ou de l'écosystème technique 200.

## Revendications

1. Dispositif (100) de modification d'un état émotionnel d'un utilisateur qui comporte :
- un lecteur (105) en temps réel de signaux électroencéphalographiques,
- un module (110) de détermination d'un état émotionnel en fonction d'un signal électroencéphalographique lu,
- un moyen (115) de détermination d'un état émotionnel cible,
- un sélecteur (120) automatique d'une succession d'au moins un fichier sonore, parmi une liste de fichiers sonores préconstituée, en fonction de l'état émotionnel cible déterminé, du signal électroencéphalographique lu et d'au moins un paramètre associé à chaque dit fichier sonore, un paramètre utilisé par le sélecteur (120) automatique pour sélectionner une succession de fichiers sonores est un indicateur représentatif d'une valeur d'état émotionnel associée à au moins un fichier sonore, la succession de fichiers sonores est configurée pour présenter un gradient croissant de valeur d'état émotionnel correspondant à l'état émotionnel cible déterminé et
- un transducteur (125) électroacoustique configuré pour lire la succession de fichiers sonore sélectionnée,
**caractérisé en ce que** le dispositif comporte, de plus :
- un sélecteur (130) secondaire d'un fichier sonore et
- un moyen (135) de mise à jour de la succession par détermination d'une nouvelle succession, postérieure à la sélection manuelle, en fonction du fichier sonore sélectionné manuellement par le sélecteur secondaire pour atteindre l'état émotionnel cible, la nouvelle succession de fichiers sonores est configurée pour présenter un gradient croissant de valeur d'état émotionnel correspondant à l'état émotionnel cible déterminé à partir de l'indicateur représentatif d'une valeur d'état émotionnel associée au fichier sonore sélectionné.

2. Dispositif (100) selon la revendication 1, qui comporte :
- un collecteur (140) d'identifiants de fichiers sonores et
- un classificateur (145) de fichier sonore configuré pour associer, à au moins un identifiant de fichier sonore, un paramètre représentatif d'un état émotionnel favorisé par ledit fichier sonore, au moins un paramètre étant choisi parmi :
- un paramètre technique, tel la durée, le mode, la tonalité, le chiffrage de la mesure ou le tempo du fichier sonore où
- un paramètre acoustique ou psychoacoustique représentatif de :
- la nature acoustique du fichier sonore, c'est-à-dire l'utilisation ou non d'instruments électroniques et/ou la proportion d'une telle utilisation,
- la nature énergisante, ou vivifiante, du fichier sonore, c'est-à-dire une mesure perceptive de l'intensité et de l'activité - les caractéristiques perceptuelles contribuant à cet attribut comprennent la gamme dynamique, l'intensité sonore perçue, le timbre, le taux d'apparition et l'entropie générale,
- la nature instrumentale du fichier sonore, c'est-à-dire l'utilisation ou non de la voix sur ledit fichier sonore,
- la nature dansante du fichier sonore, mesuré par exemple en fonction du tempo, de la stabilité du rythme, de la force du battement et de la régularité générale du fichier audio,
- la valence du fichier sonore, c'est-à-dire la positivité du fichier sonore,
- la nature de l'enregistrement du fichier sonore, c'est-à-dire l'enregistrement studio ou lors d'une prestation en directe du fichier sonore,
- la nature de densité de paroles, c'est-à-dire la proportion de mots et de musique dans un fichier sonore et/ou
- l'intensité du fichier sonore, c'est-à-dire l'intensité moyenne, mesurée en décibels, du fichier sonore.

3. Dispositif (100) selon la revendication 2, dans lequel le classificateur (145) est un système d'apprentissage automatique entraîné.

4. Dispositif (100) selon la revendication 3, dans lequel le système d'apprentissage automatique entraîné est un réseau de neurones supervisé configuré pour recevoir en guise de couche d'entrée des valeurs de paramètres et en guise de couche de sortie des indicateurs d'état émotionnel correspondant à la couche d'entrée.

5. Dispositif (100) selon l'une des revendications 3 ou 4, dans lequel le classificateur (145) est configuré pour classifier un fichier sonore en attribuant une valeur à au moins une parmi trois caractéristiques :
- la valence,
- l'excitation et
- la dominance,
au moins une caractéristique étant mise en œuvre pour déterminer un état émotionnel favorisé par une piste sonore.

6. Dispositif selon l'une des revendications 3 à 5, dans lequel le système d'apprentissage automatique est, de plus, pré-entraîné via l'utilisation d'un jeu de données non spécifique de l'utilisateur.

7. Dispositif (100) selon l'une des revendications 1 à 6, dans lequel au moins un fichier sonore est associé à un indicateur d'un comportement de l'utilisateur vis-à-vis de chaque dit fichier sonore, le sélecteur (120) automatique étant configuré pour sélectionner une succession d'au moins un fichier sonore en fonction d'une valeur dudit indicateur pour au moins un fichier sonore.

8. Dispositif (100) selon la revendication 7, dans lequel l'indicateur de comportement de l'utilisateur est un paramètre représentatif d'un nombre d'écoutes et/ou d'un nombre d'interruptions de lecture au profit d'une autre piste sonore.

9. Dispositif (100) selon l'une des revendications 7 ou 8, dans lequel le sélecteur (120) automatique comporte un filtre (121) de fichier sonore en fonction d'au moins un indicateur d'un comportement de l'utilisateur vis-à-vis d'au moins un fichier sonore, le sélecteur étant configuré pour sélectionner une succession de fichiers sonores parmi une liste de fichiers sonores filtrée par le filtre.

10. Dispositif (100) selon l'une des revendications 1 à 9, dans lequel un paramètre utilisé par le sélecteur (120) automatique pour sélectionner une succession de fichiers sonores est, de plus, un paramètre technique choisi parmi la durée, le mode, la tonalité, le chiffrage de la mesure et le tempo du fichier sonore.

11. Dispositif (100) selon l'une des revendications 1 à 10, dans lequel le lecteur (105) en temps réel de signaux électroencéphalographiques est non invasif.

12. Dispositif (100) selon la revendication 11, dans lequel le lecteur (105) est casque de type électroencéphalogramme.

## Patentansprüche

1. Die Vorrichtung (100) zur Veränderung eines emotionalen Zustands eines Benutzers, umfasst:
- einen Echtzeit-Player (105) für EEG-Signale,
- ein Modul (110) zur Bestimmung eines emotionalen Zustands anhand eines gelesenen EEG-Signals,
- ein Medium (115) zur Bestimmung eines angestrebten emotionalen Zustands,
- einen automatischen Selektor (120) für eine Abfolge von mindestens einer Audiodatei aus einer vorab erstellten Liste von Audiodateien, der sich nach dem ermittelten Ziel-Emotionszustand, dem gelesenen EEG-Signal und mindestens einem jeder Audiodatei zugeordneten Parameter richtet; ein vom automatischen Selektor (120) verwendeter Parameter zur Auswahl einer Folge von Audiodateien ist ein Indikator, der einen emotionalen Zustandswert repräsentiert, der mindestens einer Audiodatei zugeordnet ist, wobei die Folge von Audiodateien so konfiguriert ist, dass sie einen ansteigenden Gradienten des emotionalen Zustandswerts entsprechend dem bestimmten angestrebten emotionalen Zustand aufweist, und
- einen elektroakustischen Wandler (125), der so konfiguriert ist, dass die ausgewählte Folge von Audiodateien wiedergegeben wird,
wobei die Vorrichtung **dadurch gekennzeichnet ist, dass** sie außerdem Folgendes umfasst:
- einen sekundären Selektor (130) für eine Audiodatei und
- ein Medium (135) zum Aktualisieren der Reihenfolge,indem nach der manuellen Auswahl eine neue Abfolge in Abhängigkeit von der vom sekundären Selektor manuell ausgewählten Audiodatei festgelegt wird, um den angestrebten emotionalen Zustand zu erreichen, wobei die neue Folge von Audiodateien so konfiguriert ist, dass sie einen ansteigenden Gradienten des emotionalen Zustandswerts aufweist, der dem Ziel-Emotionszustand entspricht, welcher anhand des Indikators bestimmt wurde. Dieser Indikator repräsentiert einen emotionalen Zustandswert, welcher der ausgewählten Audiodatei zugeordnet ist.

2. Die Vorrichtung (100) nach Anspruch 1, umfasst Folgendes:
- einen Sammler (140) für Audiodateikennungen und
- einen Klassifizierer (145) für Audiodateien, der so konfiguriert ist, dass mindestens einer Audiodateikennung ein Parameter zugeordnet wird, der einen von der Audiodatei bevorzugten emotionalen Zustand repräsentiert, wobei mindestens ein Parameter aus folgenden Parametern ausgewählt wird:
- einem technischen Parameter, wie der Dauer, dem Modus, der Tonart, der Taktart oder dem Tempo der Audiodatei, oder
- einem akustischen oder psychoakustischen Parameter, der repräsentativ ist für:
- die akustische Beschaffenheit der Audiodatei, d. h. die Verwendung oder Nichtverwendung elektronischer Instrumente und/oder den Anteil einer solchen Verwendung,
- der energetisierenden oder belebenden Beschaffenheit der Audiodatei, d. h. einer wahrnehmbaren Messung der Intensität und Aktivität. Die Wahrnehmungsmerkmale, die zu diesem Attribut beitragen, umfassen den Dynamikbereich, die wahrgenommene Lautstärke, die Klangfarbe, die Auftrittsrate und die allgemeine Entropie,
- der instrumentale Charakter der Tondatei, d. h. die Verwendung oder Nichtverwendung von Gesang in der Audiodatei,
- der tanzbare Charakter der Audiodatei, gemessen beispielsweise anhand des Tempos, der Stabilität des Rhythmus, der Stärke des Beats und der allgemeinen Regelmäßigkeit der Audiodatei,
- die Valenz der Audiodatei, d. h. die Positivität der Audiodatei,
- die Art der Aufnahme der Audiodatei, d. h. ob es sich um eine Studioaufnahme oder eine Live-Aufnahme der Audiodatei handelt,
- die Art der Wortdichte, d. h. der Anteil von Worten und Musik in einer Audiodatei und/oder
- die Intensität der Audiodatei, d. h. die durchschnittliche Intensität der Audiodatei, gemessen in Dezibel.

3. Vorrichtung (100) nach Anspruch 2, wobei der Klassifizierer (145) ein trainiertes maschinelles Lernsystem ist.

4. Vorrichtung (100) nach Anspruch 3, wobei das trainierte maschinelle Lernsystem ein überwachtes neuronales Netzwerk ist, das so konfiguriert ist, dass es Parameterwerte als Eingangsschicht und der Eingangsschicht entsprechende Indikatoren für den emotionalen Zustand als Ausgangsschicht empfängt.

5. Vorrichtung (100) nach einem der Ansprüche 3 oder 4, wobei der Klassifizierer (145) so konfiguriert ist, dass eine Audiodatei klassifiziert wird, indem mindestens einem von drei Merkmalen ein Wert zugewiesen wird:
- der Valenz,
- der Erregung und
- der Dominanz,
wobei mindestens ein Merkmal verwendet wird, um einen durch eine Tonspur bevorzugten emotionalen Zustand zu bestimmen.

6. Vorrichtung nach einem der Ansprüche 3 bis 5, wobei das maschinelle Lernsystem zusätzlich unter Verwendung eines nicht benutzerspezifischen Datensatzes vortrainiert ist.

7. Vorrichtung (100) nach einem der Ansprüche 1 bis 6, wobei mindestens eine Audiodatei einem Indikator für ein Benutzerverhalten in Bezug auf jede Audiodatei zugeordnet ist. Der automatische Selektor (120) ist so konfiguriert, dass er eine Folge von mindestens einer Audiodatei in Abhängigkeit von einem Wert des Indikators für mindestens eine Audiodatei auswählt.

8. Vorrichtung (100) nach Anspruch 7, wobei es sich bei dem Indikator für das Benutzerverhalten um einen Parameter handelt, der eine Anzahl von Wiedergaben und/oder eine Anzahl von Unterbrechungen der Wiedergabe zugunsten einer anderen Tonspur angibt.

9. Vorrichtung (100) nach einem der Ansprüche 7 oder 8, wobei der automatische Selektor (120) einen Filter (121) für Audiodateien aufweist, der auf mindestens einen Indikator für ein bestimmtes Verhalten der Audiodatei basiert, wobei der Selektor so konfiguriert ist, dass er eine Folge von Audiodateien aus einer durch den Filter gefilterten Liste von Audiodateien auswählt.

10. Vorrichtung (100) nach einem der Ansprüche 1 bis 9, wobei ein vom automatischen Selektor (120) zur Auswahl einer Folge von Audiodateien verwendeter Parameter außerdem ein technischer Parameter ist, der aus der Dauer, dem Modus, der Tonhöhe, der Taktart und dem Tempo der Audiodatei ausgewählt wird.

11. Vorrichtung (100) nach einem der Ansprüche 1 bis 10, wobei der Echtzeit-Player (105) für EEG-Signale nicht-invasiv ist.

12. Vorrichtung (100) nach Anspruch 11, wobei der Player (105) ein EEG-Kopfhörer ist.

## Claims

1. Device (100) for modifying an emotional state of a user, which comprises:
- a real-time reader (105) of electroencephalographic signals,
- a module (110) for determining an emotional state according to an electroencephalographic signal read,
- a means (115) for determining a target emotional state,
- an automatic selector (120) for selecting a succession of at least one sound file, from a pre-compiled list of sound files, according to the target emotional state determined, the electroencephalographic signal read, and at least one parameter associated with each said sound file, a parameter used by the automatic selector (120) to select a succession of sound files being an indicator representative of an emotional state value associated to at least one sound file, the succession of sound files being configured to have an increasing emotional state value gradient corresponding to the target emotional state determined, and
- an electroacoustic transducer (125) configured to play the selected succession of sound files,
**characterised in that** the device also comprises:
- a secondary selector (130) of a sound file, and
- a means (135) for updating the succession by determining a new succession following manual selection, according to the sound file manually selected by the secondary selector to achieve the target emotional state, the new succession of sound files being configured to have an increasing emotional state value gradient corresponding to the target emotional state determined based on the indicator representative of an emotional state value associated to the sound file selected.

2. Device (100) according to claim 1, which comprises:
- a collector (140) of sound file identifiers, and
- a sound file classifier (145) configured to associate, to at least one sound file identifier, a parameter representative of an emotional state promoted by said sound file, at least one parameter being selected from:
- a technical parameter, such as the duration, mode, tonality, quantification of the beat and tempo of the sound file, or
- an acoustic or psychoacoustic parameter representative of:
- the acoustic nature of the sound file, i.e. whether electronic instruments are used or not, and/or the proportion of such a use,
- the energising, or stimulating, nature of the sound file, i.e. a perceptual measurement of the intensity and activity - the perceptual characteristics contributing to this attribute comprise the dynamic range, the perceived sound intensity, the timbre, the repetition rate and the general entropy,
- the instrumental nature of the sound file, i.e. whether or not this sound file includes voice,
- the danceable nature of the sound file, measured for example as a function of the tempo, rhythm stability, beat strength and general regularity of the audio file,
- the valence of the sound file, i.e. the positivity of the sound file,
- the recording nature of the sound file, i.e. whether the sound file contains a studio recording or a live performance recording,
- the word density nature, i.e. the proportion of words and music in a sound file, and/or
- the intensity of the sound file, i.e. the average intensity, measured in decibels, of the sound file.

3. Device (100) according to claim 2, wherein the classifier (145) is a trained machine learning system.

4. Device (100) according to claim 3, wherein the trained machine learning system is a supervised neural network configured to receive, as input layer, parameter values and, as output layer, emotional state indicators corresponding to the input layer.

5. Device (100) according to one of claims 3 or 4, wherein the classifier (145) is configured to classify a sound file by assigning a value to at least one of three characteristics:
- valence,
- energisation, and
- dominance,
at least one characteristic being utilised to determine an emotional state promoted by a sound track.

6. Device according to one of claims 3 to 5, wherein the machine learning system is also pre-trained via use of a non-specific dataset of the user.

7. Device (100) according to one of claims 1 to 6, wherein at least one sound file is associated with an indicator of a behaviour of the user with respect to each said sound file, the automatic selector (120) being configured to select a succession of at least one sound file based on a value of said indicator for at least one sound file.

8. Device (100) according to claim 7, wherein the indicator of the user's behaviour is a parameter representative of a number of listens and/or a number of plays interrupted to jump to another sound track.

9. Device (100) according to one of claims 7 or 8, wherein the automatic selector (120) comprises a sound file filter (121) based on at least one indicator of a behaviour of the user with respect to at least one sound file, the selector being configured to select a succession of sound files from a list of sound files filtered by the filter.

10. Device (100) according to one of claims 1 to 9, wherein a parameter used by the automatic selector (120) for selecting a succession of sound files is also a technical parameter selected from the duration, mode, tonality, quantification of the beat and tempo of the sound file.

11. Device (100) according to one of claims 1 to 10, wherein the real-time reader (105) of electroencephalographic signals is non-invasive.

12. Device (100) according to claim 11, wherein the reader (105) is an electroencephalogram headset.
